# EUROPÄISCHE PATENTSCHRIFT

(11) **EP 1 223 968 B1**
(45) Veröffentlichungstag und Bekanntmachung des Hinweises auf die Patenterteilung: **07.05.2003**
(21) Anmeldenummer: 00975905.1
(22) Anmeldetag: 24.10.2000
(51) Int. Cl.: A61K 38/22, A61P 13/12

(54) **VERWENDUNG VON URODILATIN ZUR BEHANDLUNG CHRONISCHER NIERENINSUFFIZIENZ MIT NIERENRESTFUNKTIONEN**
USE OF URODILATIN FOR TREATING CHRONIC KIDNEY FAILURE WITH RESIDUAL KIDNEY FUNCTIONS
UTILISATION DE L'URODILATINE POUR TRAITER UNE INSUFFISANCE RENALE CHRONIQUE AYANT DES FONCTIONS RENALES RESIDUELLES

(30) Priorität: 26.10.1999 DE 19951471
(43) Veröffentlichungstag der Anmeldung: 24.07.2002
(73) Patentinhaber: Pharis Biotec GmbH, 30625 Hannover (DE)
(72) Erfinder: FORSSMANN, Wolf-Georg, 30625 Hannover (DE); FORSSMANN, Kristin, 30175 Hannover (DE); GREB, Wolfgang, 40489 Düsseldorf (DE); MEYER, Markus, 30625 Hannover (DE)
(74) Vertreter: Meyers, Hans-Wilhelm, Dr.Dipl.-Chem.
(86) Internationale Anmeldenummer: EP0010462
(87) Internationale Veröffentlichungsnummer: WO01030376

(56) Entgegenhaltungen:
- WO-A-88/06596
- US-A- 5 691 310
- SEEMAN T ET AL: "Urinary excretion of urodilatin in healthy children and children with renal disease." PEDIATRIC NEPHROLOGY, Bd. 12, Nr. 1, Januar 1998 (1998-01), Seiten 55-59, XP000993239 ISSN: 0931-041X
- MEYER M. ET AL.: "URODILATIN, A NATRIURETIC PEPTIDE WITH CLINICAL IMPLICATIONS" EUROPEAN JOURNAL OF MEDICAL RESEARCH, Bd. 3, Februar 1998 (1998-02), Seiten 103-110, XP000993049

## Beschreibung

Gegenstand der vorliegenden Erfindung ist die Verwendung von Urodilatin zur Herstellung eines Arzneimittels zur Behandlung chronischer Niereninsuffizienz mit Nierenrestfunktionen.

Urodilatin ist ein Peptid, das Gegenstand des europäischen Patentes EP-A-0 349 545 ist. In der genannten Patentschrift werden auch Indikationen des Urodilatin genannt, bei denen Urodilatin bei Nierenversagen eingesetzt werden soll.

Patienten mit chronischer Niereninsuffizienz durchlaufen bei fortschreitender Erkrankung verschiedene Behandlungsstadien:
a) Förderung der Nierenrestfunktion,
b) Hämodialyse bzw. Peritonealdialyse und, wenn möglich, eine
c) Nierentransplantation.

In Stufe a) ist noch keine Hämodialyse oder Peritonealdialyse notwendig. Es besteht mithin das Bestreben, diese Phase auch bei negativer Prognose möglichst lange zu erhalten, um einen niereninsuffizienten Patienten möglichst lange vor der Dialyse zu verschonen. Überraschenderweise kann Urodilatin zur Verlängerung der Phase a) eingesetzt werden. Urodilatin besitzt als körpereigenes Produkt ein Wirkungsprofil synergistischer Effekt von besonderer Bedeutung: Diurese und Natriurese führen in der Niere über die Erhöhung der glomerulären Filtrationsrate (GFR) sowie Hemmung der Natrium-Rückresorption zur Flüssigkeitsausschwemmung und somit zur Ausscheidung harnpflichtiger Substanzen. Durch die gleichzeitige intrarenale Gefäßerweiterung wirkt Urodilatin nierenschützend, wie sich jetzt herausstellt.

Mithin ist Gegenstand der vorliegenden Erfindung die Verwendung des Urodilatin zur Herstellung eines Arzneimittels zur Behandlung chronischer Niereninsuffizienz mit Nierenrestfunktion.

Erfindungsgemäß kann die erfindungsgemäße Verwendung zur Verbesserung der Nierenrestfunktion bei chronisch niereninsuffizienten Patienten vor der Dialysepflicht und/oder zur Verlängerung der dialysefreien Intervalle bei chronisch niereninsuffizienten Patienten führen . Besonders die letzte Alternative ist für die betroffenen Patienten ebenfalls von großer Bedeutung, da damit die Häufigkeit der Dialyse reduziert werden kann.

In der EP-A-0 349 545 sind verschiedene klinische Indikationen angesprochen worden, bei denen Urodilatin als intravenöse Infusion eingesetzt wird. Hierzu gehört neben anderen die akute Niereninsuffizienz.

Die Menge des erfindungsgemäß zu verwendenden Urodilatin beträgt 5 ng/kg Körpergewicht bis 10 µg/kg Körpergewicht. Dem Fachmann ist es bekannt, dass diese Bereiche als Anhaltspunkte dienen zur Feststellung einer optimalen Dosierung. Es ist völlig klar, dass die geringst mögliche Menge, die zu verabreichen ist, diejenige ist, die gerade über den Schwellenwert einer feststellbaren Wirkung liegt, wohingegen die obere Grenze begrenzt ist durch auftretende toxische Erscheinungen. Typischerweise liegen die Dosierungen in dem angegebenen Bereich.

Insbesondere die Applikationen von Urodilatin als eine intravenöse Infusion in einer Dosierung von z.B. 20 ng/kg Körpergewicht/min, insbesondere über eine Zeit von 6 bis 24 Stunden bei Patienten mit einer chronischen, präterminalen, noch nicht Dialyse-pflichtigen Niereninsuffizienz führt zu einer erhöhten Ausscheidung von Flüssigkeit und harnpflichtigen Substanzen.

Darüber hinaus führt Urodilatin auch als intravenöse Infusion zwischen den Dialyse-Intervallen zu einer Stimulation der Nierenrestfunktion und somit zu einer erhöhten Ausscheidung von Flüssigkeit und harnpflichtigen Substanzen. Urodilatin eignet sich somit in besonderem Masse im Vorstadium der Dialyse zur Verbesserung der Nierenrestfunktion und führt zu einer Herauszögerung der Dialysepflicht. Außerdem eignet sich Urodilatin als Medikament zum Einsatz zwischen den Dialysen zur Verlängerung der dialysefreien Intervalle.

Die erfindungsgemäße Verwendung des Urodilatin zur Verbesserung der Ausscheidung der Flüssigkeit und harnpflichtigen Substanzen in die Bauchhöhle bei Patienten mit chronischer Niereninsuffizienz erfolgt durch Gabe des Urodilatin zum Peritonealdialysat von Patienten mit chronischer Niereninsuffizienz.

Die Therapie der chronischen, terminalen Niereninsuffizienz besteht in der Hämodialyse und in der Peritonealdialyse. Bei der Peritonealdialyse wird die Ausscheidung von Flüssigkeit und harnpflichtigen Substanzen über das in die Bauchhöhle applizierte Peritonealdialysat, das aus Zuckern und Elektrolyten besteht, gesteuert.

Überraschenderweise bewirkt die erfindungsgemäße Gabe von Urodilatin praktisch als Additiv zur Peritonealdialysat eine verstärkte Ausscheidung von Flüssigkeit und harnpflichtigen Substanzen in die Bauchhöhle. Somit kann Urodilatin auch als Medikament bei chronisch, terminalen niereninsuffizienten Patienten eingesetzt werden.

## Patentansprüche

1. Verwendung von Urodilatin zur Herstellung eines Arzneimittels zur Verbesserung von Nierenrestfunktionen bei chronisch niereninsuffizienten Patienten vor der Dialysepflicht und/oder zur Verlängerung der dialysefreien Intervalle bei chronisch niereninsuffizienten Patienten.

2. Verwendung von Urodilatin zur Herstellung eines Arzneimittels zur Verbesserung der Ausscheidung von Flüssigkeit und harnpflichtigen Substanzen in die Bauchhöhle bei Patienten mit chronischer Niereninsuffizienz indem Urodilatin zum Peritonealdialysat dieser Patienten zugefügt wird.

## Claims

1. Use of urodilatin for the preparation of a medicament for improving residual renal functions in patients suffering from chronic renal insufficiency prior to obligatory dialysis and/or for extending the dialysis-free intervals in patients suffering from chronic renal insufficiency.

2. Use of urodilatin for the preparation of a medicament for improving the secretion of liquid and of substances which are obligatorily secreted through the urine into the abdominal cavity in patients suffering from chronic renal insufficiency by adding urodilatin to the peritoneal dialysate of such patients.

## Revendications

1. Utilisation de l'urodilatine pour la fabrication d'un médicament pour l'amélioration des fonctions rénales résiduelles chez des insuffisants rénaux chroniques avant le soin par dialyse et/ou pour prolonger l'intervalle sans dialyse chez des insuffisants rénaux chroniques.

2. Utilisation de l'urodilatine pour la fabrication d'un médicament pour l'amélioration de l'élimination de liquide et de substances devant être urinées dans la cavité abdominale chez des insuffisants rénaux chroniques par addition pour ces patients d'urodilatine au dialysat péritonéal.
